# FASCICULE DE BREVET EUROPEEN

(11) **EP 1 199 066 B1**
(45) Date de publication et mention de la délivrance du brevet: **28.07.2004**
(21) Numéro de dépôt: 01402552.2
(22) Date de dépôt: 03.10.2001
(51) Int. Cl.: A61K 7/48, A61K 7/42

(54) **Composition cosmétique comprenant du rétinol et un composé filtrant le rayonnement UVA**
Kosmetisches Mittel enthaltend Retinol und einen UV-A-strahlungsfiltrierenden Agenten
Cosmetic composition containing retinol and a UV-A radiation screening compound

(30) Priorité: 17.10.2000 FR 0013285
(43) Date de publication de la demande: 24.04.2002
(73) Titulaire: L'OREAL, 75008 Paris (FR)
(72) Inventeur: Martin, Guénaelle, 75012 Paris (FR); Touzan, Philippe, 75012 Paris (FR)
(74) Mandataire: Renard, Emmanuelle

(56) Documents cités:
- EP-A- 0 641 557
- EP-A- 0 671 160
- EP-A- 0 972 511
- WO-A-98/55075

## Description

La présente invention a pour objet de nouvelles compositions cosmétiques et/ou dermatologiques, en particulier des compositions destinées au soin de la peau et comprenant, dans un milieu physiologiquement acceptable, du rétinol et un composé capable de filtrer le rayonnement UVA.

Les compositions cosmétiques et/ou dermatologiques à base de rétinoïdes ont connu un important développement au cours de ces dernières années, en particulier pour le traitement de l'acné et des imperfections cutanées, en raison de leur pouvoir kératolytique, et pour la prévention et le traitement de certains signes du vieillissement cutané intrinsèque ou photo-induit, tels que la formation de rides et la perte de fermeté et d'élasticité de la peau.

Parmi les dérivés de la famille des rétinoïdes, le rétinol, également connu sous le nom de vitamine A, présente un intérêt tout particulier. En effet, le rétinol est un constituant endogène naturel de l'organisme humain qui est bien toléré en application sur la peau jusqu'à des taux beaucoup plus élevés que l'acide rétinoïque.

Toutefois, lorsqu'il est introduit dans une composition cosmétique ou dermatologique destinée à une application topique, le rétinol se dégrade rapidement, sous l'effet de la lumière, de l'oxygène, des ions métalliques, des agents oxydants, de l'eau ou en particulier sous l'effet d'une élévation de température. La dégradation thermique du rétinol a fait l'objet d'une étude publiée dans J. Soc. Cosm. Chem. 46, 191 - 198 (July-August 1995).

La Demanderesse a par ailleurs noté que l'introduction de certains filtres UVA dans des compositions cosmétiques comprenant du rétinol occasionnait une dégradation du rétinol dans le temps, sans que les raisons de cette dégradation puissent être clairement identifiées.

Le document WO 98 55075 divulgue des compositions à base de rétinol et de filtres UV-A, notamment du type 4-t-butyl-4'-méthoxydibenzoylméthane.

Cela est particulièrement fâcheux s'agissant de compositions destinées à la prévention ou au traitement des signes cutanés du photo-vieillissement, dans la mesure où il est souvent intéressant de cumuler, dans ces compositions, les effets biologiques du rétinol, notamment sur la synthèse de collagène, aux effets des filtres solaires absorbant le rayonnement UVA. Ces filtres permettent en effet d'aider les cellules à se défendre contre l'excès de radicaux libres photo-induits et de prévenir la dégradation des fibres de collagène due aux UVA. Ils ont donc des effets anti-âge complémentaires de ceux du rétinol.

On comprend donc l'importance qui existe à disposer de compositions, notamment cosmétiques, comprenant à la fois du rétinol et un filtre UV, en particulier un filtre UVA, dans lesquelles le rétinol ne se trouve pas dégradé par le filtre.

Or, la Demanderesse a découvert avec étonnement qu'une famille donnée de filtres UVA pouvait être introduite dans des compositions comprenant du rétinol sans entraîner de dégradation de celui-ci. Ces compositions cosmétiques et/ou dermatologiques peuvent ainsi être stockées pendant plusieurs mois, sans voir se dégrader leur efficacité.

L'invention a donc pour objet une composition comprenant, dans un milieu physiologiquement acceptable, du rétinol, et un composé capable de filtrer le rayonnement UVA, caractérisée en ce que ledit composé répond à la formule (I): 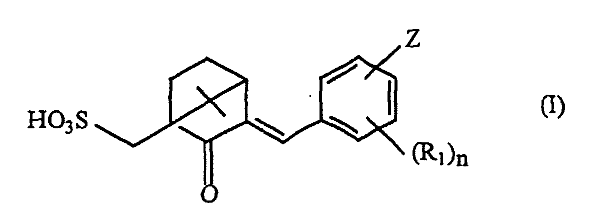 dans laquelle :
- Z désigne un groupement de formule : 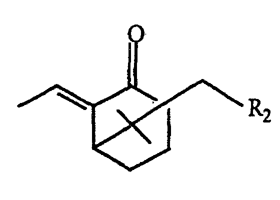
- R₂ désignant -H ou -SO₃H ;
- n désigne 0 ou un nombre entier supérieur ou égal à 1 et inférieur ou égal à 4 ;
- R1 représente un ou plusieurs radicaux alkyle ou alcoxy, identiques ou différents, linéaires ou ramifiés, contenant de 1 à 4 atomes de carbone,
- les deux radicaux méthylidène camphre se trouvant sur le noyau phényle en position méta ou para l'un par rapport à l'autre.

Eventuellement, la fonction acide sulfonique du composé de formule (I) peut être entièrement ou partiellement neutralisée par un hydroxyde de métal alcalin ou alcalino-terreux, l'ammoniaque, ou une base organique.

Les composés de formule (I) ci-dessus sont respectivement décrits dans le brevet US-4,585,597 et les demandes de brevets FR 2236515, 2282426, 2645148, 2430938 et 2592380.

Un composé de formule (I) particulièrement préféré est l'acide benzène 1,4-di(3-méthylidènecampho 10-sulfonique) de formule (II) :

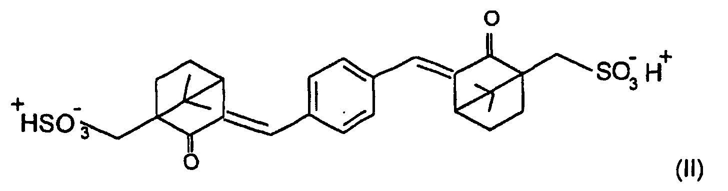

En variante, on peut utiliser dans la présente invention l'un de ses sels de métal alcalin ou alcalino-terreux, d'ammonium ou avec une base organique.

Il s'agit d'un filtre solaire capable d'absorber les rayons ultraviolets de longueur d'onde comprise entre 280 et 400 nm, avec des maxima d'absorption compris entre 320 et 400 nm, en particulier aux alentours de 345 nm.

Ce composé a également la propriété, lorsqu'il comporte au moins une fonction acide sulfonique au moins partiellement non neutralisée, de lutter contre le vieillissement intrinsèque de la peau, comme indiqué dans la demande EP-0 671 160.

Pour une meilleure protection vis-à-vis des UV, la composition selon l'invention peut, en plus du filtre UVA de formule (I) précité, renfermer au moins un composé capable de filtrer le rayonnement UVB et/ou au moins un autre composé capable de filtrer le rayonnement UVA et/ou au moins un pigment inorganique éventuellement enrobé.

Comme composé capable de filtrer le rayonnement UVB, on peut notamment citer :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, disponible auprès de la société GIVAUDAN sous la dénomination commerciale Parsol MCX ;
(3) les dérivés de β,β'-diphénylacrylate liquides, en particulier l'α-cyano-β,β' diphénylacrylate de 2-éthylhexyle, ou octocrylène, disponible auprès de la société BASF sous la dénomination commerciale UVINUL N539 ;
(4) les dérivés de l'acide p-aminobenzoïque ;
(5) le 4-méthyl benzylidène camphre disponible auprès de la société MERCK, sous la dénomination commerciale EUSOLEX 6300 ;
(6) l'acide 2-phénylbenzimidazole 5-sulfonique vendu sous la dénomination commerciale « EUSOLEX 232 » par la société MERCK
(7) les dérivés de 1,3,5-triazine, en particulier :
   - la 2,4,6-tris[4-(éthylhexyloxycarbonyl)anilino]-1,3,5-triazine, disponible auprès de la société BASF sous la dénomination commerciale UVINUL T150, et
   - le composé répondant à la formule suivante : 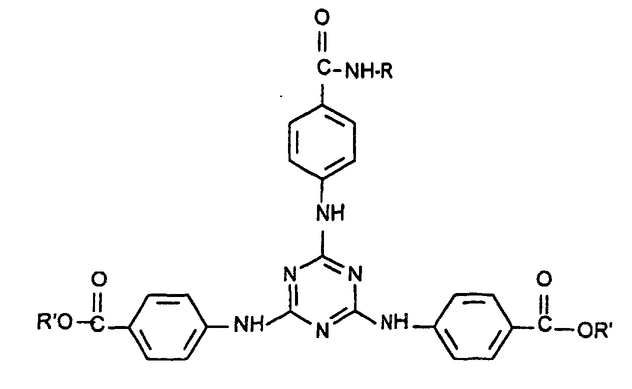
   dans laquelle R' désigne un radical éthyl-2 hexyle et R désigne un radical tert- butyle, disponible auprès de la société SIGMA 3V sous la dénomination commerciale UVASORB HEB ;
(8) leurs mélanges.

Comme autre composé capable de filtrer le rayonnement UVA, on peut notamment citer :
(1) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), disponible auprès de la société BASF sous la dénomination commerciale UVINUL M40 ;
   - l'acide 2-hydroxy-4-méthoxy-benzophénone-5-sulfonique (benzophénone-4) ainsi que sa forme sulfonate (benzophénone-5), disponible auprès de la société BASF sous la dénomination commerciale UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12), les benzophénones 3 et 5 étant préférées ;
(2) les dérivés de triazine, et en particulier la 2,4-bis [4-(2-éthyl-hexyloxy)-2-hydroxyphényl]-6-(4-méthoxy-phényl)-1,3,5-triazine disponible auprès de la société CIBA GEIGY sous la dénomination commerciale TINOSORB S et le 2,2'-méthylènebis-[6-(2H benzotriazol-2-yl)4-(1,1,3,3-tétraméthylbutyl)phénol] disponible auprès de la société CIBA GEIGY sous la dénomination commerciale TINOSORB M;
(3) leurs mélanges.

Par "pigments inorganiques éventuellement enrobés", on entend notamment les nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

La composition selon l'invention est de préférence destinée à un usage cosmétique ou dermatologique, avantageusement cosmétique. Elle est destinée à une application topique et contient donc généralement un milieu physiologiquement acceptable, c'est-à-dire compatible avec la peau.

La composition selon l'invention comprend en général une quantité de rétinol efficace pour obtenir l'effet recherché, par exemple comprise entre 0,01 et 0,2% en poids, et de préférence comprise entre 0,01 et 0,15 % en poids, par rapport au poids total de la composition. Elle renferme en outre une quantité de filtre UVA de formule (I) suffisante pour lui conférer le Facteur de Protection Solaire voulu, par exemple de 0,5 à 5% en poids, et de préférence de 0,7 à 3% en poids, de filtre UVA de formule (I), par rapport au poids total de la composition.

Cette composition peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), d'une émulsion triple (E/H/E ou H/E/H) ou d'une dispersion vésiculaire de type ionique et/ou non ionique. Ces compositions sont préparées selon les méthodes usuelles. De préférence, la composition selon l'invention est sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, en particulier sous forme de stick pour les lèvres. Elle peut être utilisée comme produit de soin et/ou comme produit de maquillage pour la peau.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association selon l'invention.

Lorsque la composition de invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La présente invention concerne également les utilisations cosmétiques de la composition décrite précédemment, notamment pour prévenir ou traiter les signes du vieillissement cutané intrinsèque ou photo-induit.

L'invention sera mieux comprise, et ses avantages ressortiront mieux, à la lumière des exemples suivants, qui sont donnés à titre illustratif.

### EXEMPLES

### Exemple 1 : Composition cosmétique

| *Phase A* | |
|---|---|
| Stéarate de glycéryle et stéarate de PEG-100 | 2,1 % |
| Polysorbate 60 | 0,9 % |
| Alcool cétylique | 2,6 % |
| Polyisobutène hydrogéné | 12 % |
| Hexyldécanol | 8 % |
| BHT | 0,1 % |
| Conservateur | 0,15 % |
| | |

| *Phase B* | |
|---|---|
| Eau | qsp 100 % |
| Glycérine | 3 % |
| Conservateur | 0,55 % |
| Sel pentasodique d'acide éthylenediamine tétraméthylène | |
| phosphonique | 0,07% |
| | |

| *Phase C* | |
|---|---|
| Gomme de xanthane | 0,1 % |
| Carbomer | 0,4 % |

| *Phase D* | |
|---|---|
| Eau | 5 % |
| Triéthanolamine | 0,38 % |
| | |

| *Phase* E | |
|---|---|
| Acide benzène 1,4-di(3-méthylidène-10-camphosulfonique) | 1 % |
| Triéthanolamine | 0,19 % |
| | |

| *Phase F* | |
|---|---|
| Rétinol | 0,1 % |

La composition ci-dessus peut être préparée de la manière suivante.

Les phases A et B sont chauffées séparément sous agitation à 75°C jusqu'à parfaite solubilisation. Les phases D et E sont préparées séparément sous agitation à température ambiante. La phase A est ensuite transvasée dans la phase B sous agitation à 75°C pendant 5-10 min, puis l'ensemble est refroidi à 50°C. Les constituants de la phase C sont ensuite introduits dans le mélange des phases A et B sous agitation à 50°C, et après homogénéisation complète, on ajoute successivement les phases D et E sous agitation. Le mélange est refroidi à température ambiante. Après réalisation d'un inertage à l'azote, la phase F est introduite en cuve sous agitation.

Cette composition peut être utilisée quotidiennement comme crème de jour pour prévenir et lutter contre les rides et raffermir la peau.

### Exemple 2 : Mise en évidence de la stabilisation du rétinol

La stabilité du rétinol a été évaluée dans les trois compositions A à C ci-après :
- Composition A :: Composition de l'Exemple 1 sans la phase E
- Composition B :: Composition de l'Exemple 1
- Composition C :: Composition de l'Exemple 1 sans la phase E et avec 1% de 4-tert-butyl-4'-méthoxydibenzoylméthane (commercialisé sous la dénomination Parsol 1789 par la société GIVAUDAN) dans la phase A.

Les quantités pondérales de rétinol respectivement présentes dans les compositions A à C ci-dessus ont été déterminées, d'une part, à température ambiante, immédiatement après préparation (To) de ces compositions, et, d'autre part, après deux mois de conservation à 45°C (T2m).

Ces mesures ont été effectuées par Chromatographie Liquide Haute Pression, en utilisant comme étalon une solution de rétinol à 16 µg/ml dans le THF, préparée à partir d'un échantillon de Rétinol 10S commercialisé par la société BASF sous la forme d'une solution huileuse à 10% de rétinol.

Les résultats obtenus sont les suivants :

| | To | T2m |
|---|---|---|
| Composition A | 0,108 % | 0,102 % |
| Composition B | 0,108 % | 0,097 % |
| Composition C | 0,105 % | 0,071 % |

Compte tenu de la précision de la méthode de dosage utilisée, on peut en déduire que la composition B comprenant, comme filtre UVA, l'acide benzène 1,4-di(3-méthylidène-10-camphosulfonique), est aussi stable que la composition A ne contenant pas de filtre UV et nettement plus stable que la composition C comprenant un autre filtre UVA.

## Revendications

1. Composition comprenant, dans un milieu physiologiquement acceptable, du rétinol, et un composé capable de filtrer le rayonnement UVA, **caractérisée en ce que** ledit composé répond à la formule (I): 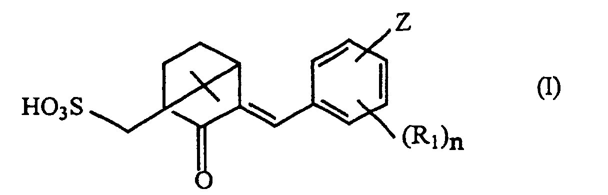 dans laquelle :
- Z désigne un groupement de formule : 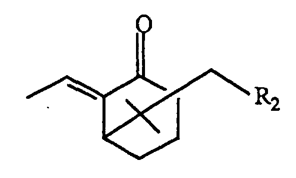
- R₂ désignant -H ou -SO₃H ;
- n désigne 0 ou un nombre entier supérieur ou égal à 1 et inférieur ou égal à 4 ;
- R1 représente un ou plusieurs radicaux alkyle ou alcoxy, identiques ou différents, linéaires ou ramifiés, contenant de 1 à 4 atomes de carbone,
- les deux radicaux méthylidène camphre se trouvant sur le noyau phényle en position méta ou para l'un par rapport à l'autre,
dont la fonction acide sulfonique peut éventuellement être entièrement ou partiellement neutralisée par un hydroxyde de métal alcalin ou alcalino-terreux, l'ammoniaque, ou une base organique.

2. Composition selon la revendication 1, **caractérisée en ce que** le composé de formule (I) est l'acide benzène 1,4-di(3-méthylidène-10-camphosulfonique) ou l'un de ses sels de métal alcalin ou alcalino-terreux, d'ammonium ou avec une base organique.

3. Composition selon la revendication 1 ou 2, **caractérisée en ce qu'**elle renferme de 0,01 à 0,15% en poids de rétinol, par rapport au poids total de la composition.

4. Composition selon l'une quelconque des revendications 1 à 3, **caractérisée en ce qu'**elle renferme de 0,7 à 3% en poids de composé de formule (I), par rapport au poids total de la composition.

5. Composition selon l'une quelconque des revendications 1 à 4, **caractérisée en ce qu'**elle est sous la forme d'une émulsion huile-dans-eau.

6. Composition selon l'une quelconque des revendications 1 à 5, **caractérisée en ce qu'**elle renferme en outre au moins un composé capable de filtrer le rayonnement UVB et/ou au moins un autre composé capable de filtrer le rayonnement UVA et/ou au moins un pigment inorganique éventuellement enrobé.

7. Composition selon la revendication 6, **caractérisée en ce que** ledit composé capable de filtrer le rayonnement UVB est choisi parmi :
(1) les dérivés de l'acide salicylique, en particulier le salicylate d'homomenthyle et le salicylate d'octyle ;
(2) les dérivés de l'acide cinnamique,
(3) les dérivés de β,β'-diphénylacrylate liquides,
(4) les dérivés de l'acide p-aminobenzoïque ;
(5) le 4-méthyl benzylidène camphre ;
(6) l'acide 2-phénylbenzimidazole 5-sulfonique ;
(7) les dérivés de 1,3,5-triazine,
(8) leurs mélanges.

8. Composition selon la revendication 6, **caractérisée en ce que** ledit composé capable de filtrer le rayonnement UVB est choisi parmi :
(1) le salicylate d'homomenthyle, et le salicylate d'octyle,
(2) le p-méthoxycinnamate de 2-éthylhexyle,
(3) l'α-cyano-β,β'-diphénylacrylate de 2-éthylhexyle, ou octocrylène,
(4) le 4-méthylbenzylidène camphre,
(5) l'acide 2-phénylbenzylimidazole-5-sulfonique,
(6) la 2,4,6-tris[4-(éthylhexyloxycarbonyl)anilino]-1,3,5-triazine,
(7) le composé répondant à la formule suivante : 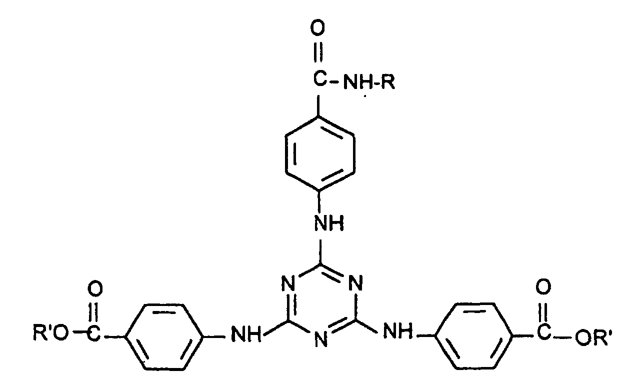
dans laquelle R' désigne un radical éthyl-2-hexyle et R désigne un radical tert- butyle.

9. Composition selon la revendication 6, 7 ou 8 **caractérisée en ce que** ledit autre composé capable de filtrer le rayonnement UVA est choisi parmi :
(1) les dérivés de benzophénone, en particulier la 2-hydroxy-4-méthoxy-benzophénone (benzophénone-3) et l'acide 2-hydroxy-4-méthoxy-benzophénone-5-sulfonique ;
(2) les dérivés de triazine, et en particulier la 2,4-bis [4-(2-éthyl-hexyloxy)-2-hydroxyphényl]-6-(4-méthoxy-phényl)-1,3,5-triazine et le 2,2'-méthylènebis-[6-(2H benzotriazol-2-yl)4-(1,1,3,3-tétraméthylbutyl)phénol] ; et
(3) leurs mélanges.

10. Composition selon l'une quelconque des revendications 6 à 9 **caractérisée en ce que** ledit autre composé capable de filtrer le rayonnement UVA est choisi parmi :
(1) la 2-hydroxy-4-méthoxy-benzophénone (benzophénone-3) et l'acide 2-hydroxy-4-méthoxy-benzophénone-5-sulfonique,
(2) la 2,4-bis [4-(2-éthyl-hexyloxy)-2-hydroxyphényl]-6-(4-méthoxy-phényl)-1,3,5-triazine et le 2,2'-méthylènebis-[6-(2H benzotriazol-2-yl)4-(1,1,3,3-tétraméthylbutyl)phénol].

11. Composition selon la revendication 6, **caractérisée en ce que** ledit pigment inorganique est choisi parmi les nanopigments d'oxyde de titane, de fer, de zinc, de zirconium ou de cérium éventuellement enrobés d'alumine et/ou de stéarate d'aluminium.

12. Utilisation cosmétique de la composition selon l'une quelconque des revendications 1 à 11, pour prévenir ou traiter les signes du vieillissement cutané intrinsèque ou photo-induit.

## Patentansprüche

1. Zusammensetzung, die in einem physiologisch verträglichen Medium Retinol und eine Verbindung enthält, die die UV-A-Strahlung ausfiltern kann, **dadurch gekennzeichnet, dass** die Verbindung der folgenden Formel (I) entspricht: 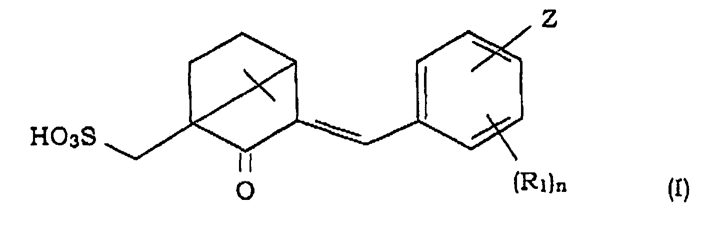 worin bedeuten:
- Z eine Gruppe der Formel: 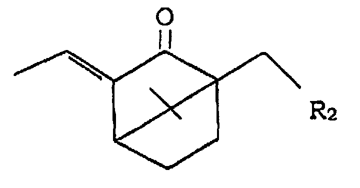
- R₂ -H oder -SO₃H;
- n 0 oder eine ganze Zahl größer oder gleich 1 und kleiner oder gleich 4;
- R₁ bedeutet eine oder mehrere Alkyl- oder Alkoxygruppen, die gleich oder verschieden sind und geradkettig oder verzweigt vorliegen und 1 bis 4 Kohlenstoffatome enthalten;
- die beiden Methylidencamphergruppen befinden sich an dem Phenylring in Bezug auf die jeweils andere Gruppe in metaoder para-Stellung,
wobei die Sulfonsäurefunktion gegebenenfalls ganz oder teilweise mit einem Alkalihydroxid, Erdalkalihydroxid, Ammoniak oder einer organischen Base neutralisiert sein kann.

2. Zusammensetzung nach Anspruch 1, **dadurch gekennzeichnet, dass** es sich bei der Verbindung der Formel (I) um die Benzol-1,4-di-(3-methyliden-10-camphersulfonsäure) oder eines ihrer Alkalimetallsalze, Erdalkalimetallsalze, Ammoniumsalze oder eines ihrer Salze mit einer organischen Base handelt.

3. Zusammensetzung nach Anspruch 1 oder 2, **dadurch gekennzeichnet, dass** sie 0,01 bis 0,15 Gew.-% Retinol, bezogen auf das Gesamtgewicht der Zusammensetzung, enthält.

4. Zusammensetzung nach einem der Ansprüche 1 bis 3, **dadurch gekennzeichnet, dass** sie 0,7 bis 3 Gew.-% der Verbindung der Formel (I), bezogen auf das Gesamtgewicht der Zusammensetzung, enthält.

5. Zusammensetzung nach einem der Ansprüche 1 bis 4, **dadurch gekennzeichnet, dass** sie als Öl-in-Wasser-Emulsion vorliegt.

6. Zusammensetzung nach einem der Ansprüche 1 bis 5, **dadurch gekennzeichnet, dass** sie ferner mindestens eine Verbindung, die die UV-B-Strahlung ausfiltern kann, und/oder mindestens eine weitere Verbindung, die die UV-A-Strahlung ausfiltern kann, und/oder mindestens ein gegebenenfalls umhülltes, organisches Pigment enthält.

7. Zusammensetzung nach Anspruch 6, **dadurch gekennzeichnet, dass** die Verbindung, die die UV-B-Strahlung ausfiltern kann, unter den folgenden Verbindungen ausgewählt ist:
(1) Salicylsäurederivaten, insbesondere Homornenthylsalicylat und Octylsalicylat;
(2) Zimtsäurederivaten;
(3) flüssigen β,β'-Diphenylacrylatderivaten
(4) p-Aminobenzoesäurederivaten;
(5) 4-Methylbenzylidencampher;
(6) 2-Phenylbenzimidazol-5-sulfonsäure;
(7) 1,3,5-Triazinderivaten und
(8) deren Gemischen.

8. Zusammensetzung nach Anspruch 6, **dadurch gekennzeichnet, dass** die Verbindung, die die UV-B-Strahlung ausfiltern kann, unter den folgenden Verbindungen ausgewählt ist:
(1) Hornomenthylsalicylat und Octylsalicylat;
(2) 2-Ethylhexyl-p-methoxycinnamat;
(3) 2-Ethylhexyl-α-Cyano-β,β'-diphenylacrylat oder Octocrylen;
(4) 4-Methylbenzylidencampher;
(5) 2-Phenylbenzimidazol-5-sulfonsäure;
(6) 2,4,6-Tris[4-(ethylhexyloxycarbonyl)anilino]-1,3,5,-triazin;
(7) der Verbindung der folgenden Formel: 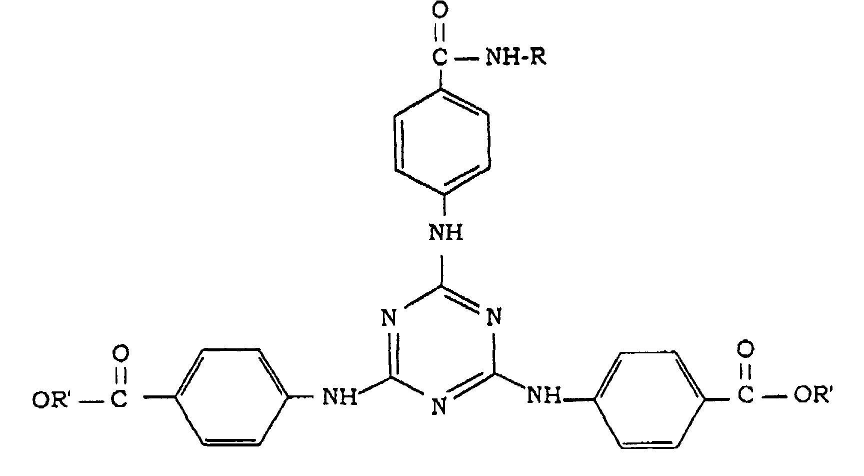
worin R' eine 2-Ethylhexylgruppe bedeutet und R die *t*-Butylgruppe ist.

9. Zusammensetzung nach Anspruch 6, 7 oder 8, **dadurch gekennzeichnet, dass** die weitere Verbindung, die die UV-A-Strahlung ausfiltern kann, unter den folgenden Verbindungen ausgewählt ist:
(1) Benzophenonderivaten, insbesondere 2-Hydroxy-4-methoxybenzophenon (Benzophenon-3) und 2-Hydroxy-4-methoxybenzophenon-5-sulfonsäure;
(2) Triazinderivaten, insbesondere 2,4-Bis[4-(2-ethyl-hexyloxy)-2-hydroxyphenyl]-6-(4-methoxy-phenyl) 1,3,5-triazin und 2,2'-Methylen-bis[6-(2H-benzotriazol-2-yl)-4-(1,1,3,3-tetramethylbutyl)phenol]; und
(3) deren Gemischen.

10. Zusammensetzung nach einem der Ansprüche 6 bis 9, **dadurch gekennzeichnet, dass** die weitere Verbindung, die die UV-A-Strahlung ausfiltern kann, unter den folgenden Verbindungen ausgewählt ist:
(1) 2-Hydroxy-4-methoxy-benzophenon (Benzophenon-3) und 2-Hydroxy-4-methoxy-benzophenon-5-sulfonsäure; und
(2) 2,4-Bis[4-(2-ethyl-hexyloxy)-2-hydroxyphenyl]-6-(4-methoxyphenyl)-1,3,5-triazin und 2,2'-Methylen-bis[6-(2H-benzotriazol-2-yl)-4-(1,1,3,3-tetramethylbutyl)-phenol].

11. Zusammensetzung nach Anspruch 6, **dadurch gekennzeichnet, dass** das anorganische Pigment unter den Nanopigmenten der Oxide von Titan, Eisen, Zink, Zirkonium oder Cer ausgewählt ist, die gegebenenfalls mit Aluminiumoxid und/oder Aluminiumstearat umhüllt sind.

12. Kosmetische Verwendung der Zusammensetzung nach einem der Ansprüche 1 bis 11 zur Vorbeugung oder Behandlung der Anzeichen der altersbedingten oder lichtinduzierten Hautalterung.

## Claims

1. Composition comprising, in a physiologically acceptable medium, retinol and a compound capable of screening out UVA radiation, **characterized in that** the said compound corresponds to the formula (I) : 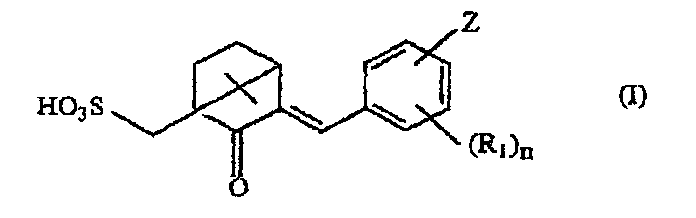 in which
- Z denotes a group of formula: 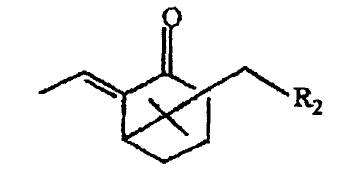
- R₂ denoting -H or -SO₃H;
- n denotes 0 or an integer of greater than or equal to 1 and less than or equal to 4;
- R₁ represents one or more identical or different and linear or branched alkyl or alkoxy radicals comprising from 1 to 4 carbon atoms,
- the two methylidenecamphor radicals being found on the phenyl nucleus in the meta or para position with respect to one another,
the sulphonic acid functional group of which can optionally be entirely or partially neutralized by an alkali metal or alkaline earth metal hydroxide, ammonia or an organic base.

2. Composition according to Claim 1, **characterized in that** the compound of formula (I) is benzene-1,4-di(3-methylidene-10-camphorsulphonic acid) or one of its alkali metal, alkaline earth metal or ammonium salts or one of its salts with an organic base.

3. Composition according to Claim 1 or 2, **characterized in that** it includes from 0.01 to 0.15% by weight of retinol, with respect to the total weight of the composition.

4. Composition according to any one of Claims 1 to 3, **characterized in that** it includes from 0.7 to 3% by weight of compound of formula (I), with respect to the total weight of the composition.

5. Composition according to any one of Claims 1 to 4, **characterized in that** it is in the form of an oil-in-water emulsion.

6. Composition according to any one of Claims 1 to 5, **characterized in that** it additionally includes at least one compound capable of screening out UVB radiation and/or at least one other compound capable of screening out UVA radiation and/or at least one optionally coated inorganic pigment.

7. Composition according to Claim 6, **characterized in that** the said compound capable of screening out UVB radiation is chosen from:
(1) salicylic acid derivatives, in particular homomenthyl salicylate and octyl salicylate;
(2) cinnamic acid derivatives;
(3) liquid β,β'-diphenylacrylate derivatives;
(4) p-aminobenzoic acid derivatives;
(5) 4-methylbenzylidenecamphor;
(6) 2-phenylbenzimidazole-5-sulphonic acid;
(7) 1,3,5-triazine derivatives;
(8) their mixtures.

8. Composition according to Claim 6,
**characterized in that** the said compound capable of screening out UVB radiation is chosen from:
(1) homomenthyl salicylate and octyl salicylate;
(2) 2-ethylhexyl p-methoxycinnamate;
(3) 2-ethylhexyl α-cyano-β,β'-diphenylacrylate, or octocrylene;
(4) 4-methylbenzylidenecamphor;
(5) 2-phenylbenzimidazole-5-sulphonic acid;
(6) 2,4,6-tris[4-(ethylhexyloxycarbonyl)anilino]-1,3,5-triazine;
(7) the compound corresponding to the following formula: 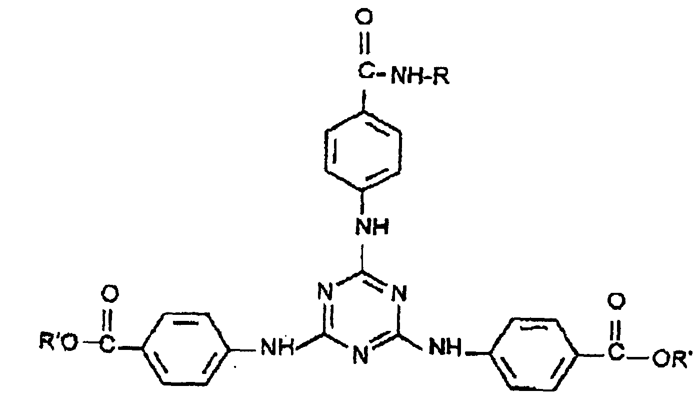
in which R' denotes a 2-ethylhexyl radical and R denotes a tert-butyl radical.

9. Composition according to Claim 6, 7 or 8, **characterized in that** the said other compound capable of screening out UVA radiation is chosen from:
(1) benzophenone derivatives, in particular 2-hydroxy-4-methoxybenzophenone (benzophenone-3) and 2-hydroxy-4-methoxybenzophenone-5-sulphonic acid;
(2) triazine derivatives and in particular 2,4-bis(4-(2-ethylhexyloxy)-2-hydroxyphenyl]-6-(4-methoxyphenyl)-1,3,5-triazine and 2,2'-methylenebis[6-(2H-benzotriazol-2-yl)-4-(1,1,3,3-tetramethylbutyl)phenol]; and
(3) their mixtures.

10. Composition according to any one of Claims 6 to 9, **characterized in that** the said other compound capable of screening out UVA radiation is chosen from:
(1) 2-hydroxy-4-methoxybenzophenone (benzophenone-3) and 2-hydroxy-4-methoxybenzophenone-5-sulphonic acid;
(2) 2,4-bis[4-(2-ethylhexyloxy)-2-hydroxyphenyl]-6-(4-methoxyphenyl)-1,3,5-triazine and 2,2'-methylenebis[6-(2H-benzotriazol-2-yl)-4-(1,1,3,3-tetramethylbutyl)phenol].

11. Composition according to Claim 6, **characterized in that** the said inorganic pigment is chosen from titanium oxide, iron oxide, zinc oxide, zirconium oxide or cerium oxide nanopigments, optionally coated with alumina and/or with aluminium stearate.

12. Cosmetic use of the composition according to any one of Claims 1 to 11 for preventing or treating signs of intrinsic or photoinduced cutaneous ageing.
